# EUROPEAN PATENT APPLICATION

(11) **EP 3 944 834 A1**
(43) Date of publication of application: **02.02.2022**
(21) Application number: 20188588.6
(22) Date of filing: 30.07.2020
(51) Int. Cl.: A61B 34/20, A61B 5/06, A61B 34/00, A61B 1/00, A61B 5/00, A61B 34/10, A61B 90/00, A61B 17/00

(54) **NAVIGATION OPERATION INSTRUCTIONS**

(71) Applicant: Koninklijke Philips N.V., 5656 AG Eindhoven (NL)
(72) Inventor: BUIL, Vincentius Paulus, 5656 AE Eindhoven (NL); MITCHELL, Andrew David, 5656 AE Eindhoven (NL); KLEIN TEESELINK, Ina, 5656 AE Eindhoven (NL); LUI, Wing Lam, 5656 AE Eindhoven (NL)
(74) Representative: Philips Intellectual Property & Standards

(57) **Abstract**

The present invention relates to providing navigation operation instructions, for example during catheterization. In order to provide improved navigation instructions, a device (10) for in-body navigation operation instructions is provided. The device comprises a position data supply (12), an anatomical data supply (14), a processor (16) and an output (18). The position data supply is configured to provide current position data of a tool portion with a tool tip inserted in an anatomy structure in a region of interest of a subject. The anatomical 3D data supply is configured to provide 3D information of the anatomy structure; and to provide target information for the tool tip movement inside the anatomy structure. The processor is configured to determine a current location of the tool tip in the anatomy structure based on the current position data; to determine at least one of the group of movement specification and the trajectory information based on the target information and the determined current location in order to align the movement of the tool tip with the target information; and, based on the at least one of the group of the movement specification and the trajectory information, to determine operation instructions for the user to achieve a movement of the tool tip according to the at least one of the group of the movement specification and the trajectory information. The output is configured to provide the operation instructions to the user.

## Description

### FIELD OF THE INVENTION.

The present invention relates to providing navigation operation instructions, for example during catheterization. The present invention relates in particular to a device for in-body navigation operation instructions, to a catheter system and to a method for providing in-body navigation operation instructions.

### BACKGROUND OF THE INVENTION

Catheterization is a medical procedure used to examine, diagnose and treat certain patient conditions in a minimally invasive way. To get access to the region of interest, a catheter - a thin, hollow tube made of medical-grade materials - of a smaller diameter than the vessel/artery of interest is inserted into a blood vessel/artery, starting from the patient's arm (radial artery), leg (femoral artery/vein), groin (upper thigh), or neck (internal jugular vein) and is threaded into the patient's region of interest. Each blood vessel has its advantages and disadvantages. By using the catheter, the doctor can do diagnostic tests and treatments on a patient's region of interest. Catheters come in numerous shapes, lengths, diameters and other special features such as electrodes and balloons. Once in place, they are used, for example, to perform measurements or an intervention. In case of a cardiac catheterization for example, the catheter is guided into the patient's heart. As an example, the term catheterization refers to cardiac catheterization (also known as heart cath).

As an example, catheterization procedures can be performed in special laboratories with fluoroscopy and highly maneuverable patient tables. These *"cath labs"* can be equipped with cabinets of catheters, stents, balloons, etc. of various sizes to increase operational efficiency during the intervention.

It has been shown that visualization is an important aspect for catheterization, as doctors must be able to see blockages in the arteries. It commonly includes fluoroscopy, but can also include forms of echocardiography (TTE, TEE, ICE) or ultrasound (IVUS). For example, ultrasound uses sound waves to create detailed pictures of the heart's blood vessels. Monitors in a cath-lab may show the catheter in the anatomy as the operation is in progress, e.g. live fluoroscopy images, ECG, pressure waves etc.

Navigating through the vessels to access the heart is a challenge during the procedure because anatomy of individuals is different and complex. As an example, WO 2014/100530 A1 relates to catheter steering. In some examples, surgeons employ X-ray images taken using contrast fluids to locate the vessels, while feeding the catheter. Therefore, they monitor the catheter's position on the monitor by taking X-ray images when needed and guide it through the vessels by turning the tip to maneuver through junctions or through the anatomy of the heart. Translational movements are achieved by pushing the catheter. The maneuvering of the tip is performed through clock (roll), yaw and pitch movements to turn the tip to maneuver through junctions or the anatomy of the heart. As an example, by rolling the catheter clockwise or counter-clockwise, the tip of the catheter changes direction. The lack of 3D information, however, makes the procedure challenging in terms of avoiding to create damage, hence also time consuming, because of the extra effort the surgeons have to spend to mitigate that risk.

### SUMMARY OF THE INVENTION

There may thus be a need to provide improved navigation instructions. The object of the present invention is solved by the subject-matter of the independent claims; further embodiments are incorporated in the dependent claims. It should be noted that the following described aspects of the invention apply also for the device for in-body navigation operation instructions, for the catheter system and for the method for providing in-body navigation operation instructions.

According to the present invention, a device for in-body navigation operation instructions is provided. The device comprises a position data supply, an anatomical data supply, a processor and an output. The position data supply is configured to provide current position data of a tool portion with a tool tip inserted in an anatomy structure in a region of interest of a subject. The anatomical 3D data supply is configured to provide 3D information of the anatomy structure. The anatomical 3D data supply is also configured to provide target information for the tool tip movement inside the anatomy structure. The processor is configured to determine a current location of the tool tip in the anatomy structure based on the current position data. The processor is also configured to determine at least one of the group of movement specification and trajectory information based on the target information and the determined current location in order to align the movement of the tool tip with the target information. The processor is further configured, based on the at least one of the group of the movement specification and the trajectory information, to determine operation instructions for the user to achieve a movement of the tool tip according to the at least one of the group of the movement specification and the trajectory information. The output is configured to provide the operation instructions to the user.

This provides the effect that the surgeon, operating the device on his/her own, is provided with major help in regards to what specific moves should exactly be done next in order to get around junctions: what controls to move in what direction to what extend to make efficient turns, where they can move faster, where they better slow down, etc. In other words, valuable operational guidance on navigating the catheter in 3D through the vessel structures is provided. In particular for complex and fine vessel structures such as in the lungs, route finding and maneuvering to the desired region of interest, being non-trivial and risky tasks, are facilitated.

As an example, the processor is configured to determine movement specification based on the target information and the determined current location in order to align the movement of the tool tip with the target information. The processor is further configured, based on at the movement specification, to determine operation instructions for the user to achieve a movement of the tool tip according to the movement specification.

As another example, the processor is also configured to determine trajectory information based on the target information and the determined current location in order to align the movement of the tool tip with the target information. The processor is further configured, based on the trajectory information, to determine operation instructions for the user to achieve a movement of the tool tip according to the trajectory information. In an example, the tool is a catheter and the tool tip is a catheter tip. The anatomy structure is a vascular structure.

In an example, data representative of a graphical representation of at least the anatomy structure of the region of interest is provided and the processor is configured to generate image data showing the tool tip in its current position in context of the anatomy structure. The output is a display configured to show the image data comprising the tool tip and the anatomy structure in combination with the operation instructions.

In an example, the operation instructions comprise instructions for the user for handling a control handle of a tool, to which tool the tool tip is attached to, which tool tip is configured to be maneuvered within the anatomy structure of the region of interest of a subj ect.

As an option, the operation instructions are provided as handling instructions for the user how to operate a plurality of operating controls of the control handle of the tool, wherein the operating controls relate to movement direction of the tip, bending of the tip portion and movement vector of the tip.

As an effect, the operating instructions are translated according to the view provided to the user.

In an example, the plurality of operating controls is provided with graphic indicators visible at least for the user of the handle. The operation instructions relate to the graphic indicators. For visual differentiation, the graphic indicators can comprise at least one of the group of colors, patterns and forms.

This provides an intuitive way for the user, since the operation instructions can be applied directly without the need for a transfer from the output to the respective spatial arrangement of the control handle.

In an example, it is further provided a control handle interface, which is configured to receive handle information relating to the operating controls of the control handle and to provide same to the processor. The processor is further configured to determine the operation instructions based on the handle information.

In an example, the control handle interface is configured to receive position data of the handle comprising at least one of the group of (clocking rotation) orientation in space, location in relation to a subject, and distance between control handle and catheter tip. The processor is further configured to determine the operation instructions based on at least one of the group of orientation in space, location in relation to the subject, and distance between control handle and catheter tip.

As an example, the catheter is configured for feeding through the control handle. As another example, the control handle is configured to be fixed to the end of the catheter. Dependent on how much of the catheter has entered the body, it is more or less subject to friction from the anatomy, thus the catheter tip may respond less directly to clocking of the control handle. This may also be the case for the bending of the catheter tip in relation to control input. In an example, the operational instructions of how much clocking or bending should be applied at the control handle, is adjusted accordingly taking these location dependent deviations into account.

In an example, the operation instructions are provided as augmented reality by overlaying the instructions on the physical control handle via a head-mounted display. Alternatively, the operation instructions are overlayed on a virtual representation of the handle, shown on a stationary display.
As an option, in addition to the operation instructions, navigation information is indicated, wherein the operation instructions are decoupled from the navigation information.

According to the present invention, also a catheter system is provided. The system comprises a catheter comprising a control handle, a catheter body and a catheter tip. The system comprises a device for catheterization operation instructions according to one of the preceding examples. The catheter tip is attached to one end of the catheter body and the catheter control handle is connected to the catheter body towards the other end of the catheter body. The operation instructions comprise instructions for user handling of the control handle in order to maneuver the catheter tip when inserted in a vascular structure of a region of interest of a subject.

According to the present invention, also a method for providing in-body navigation operation instructions is provided. The method comprises the following steps:
- providing current position data of an anatomy portion with a tool tip inserted in an anatomy structure in a region of interest of a subject;
- providing 3D information of the anatomy structure;
   providing target information for the anatomy tip movement inside the anatomy structure;
- determining a current location of the anatomy tip in the anatomy structure based on the current position data;
- determining at least one of the group of movement specification and trajectory information based on the target information and the determined current location in order to align the movement of the anatomy tip with the target information;
- determining, based on the at least one of the group of the movement specification and the trajectory information, operation instructions for the user to achieve a movement of the anatomy tip according to the at least one of the group of the movement specification and the trajectory information; and
- providing the operation instructions to the user.

According to an aspect, for navigating a tool at least partly inserted inside a subject, the user is provided with operation instructions that will then result in the desired navigation result. The operation instructions thus comprise handling instructions for the user in a way that is adapted to the user's handling situation, e.g. to the handle used by the user, such that navigation is facilitated.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for in-body navigation operation instructions.
Fig. 2 shows basic method steps of an example of a method for providing in-body navigation operation instructions.
Fig. 3a and Fig. 3b show examples of in-body navigation operation instructions presented to a user.
Fig. 4 shows an example of a catheter system.
Fig. 5 shows a further example of a workflow in relation with providing in-body navigation operation instructions.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

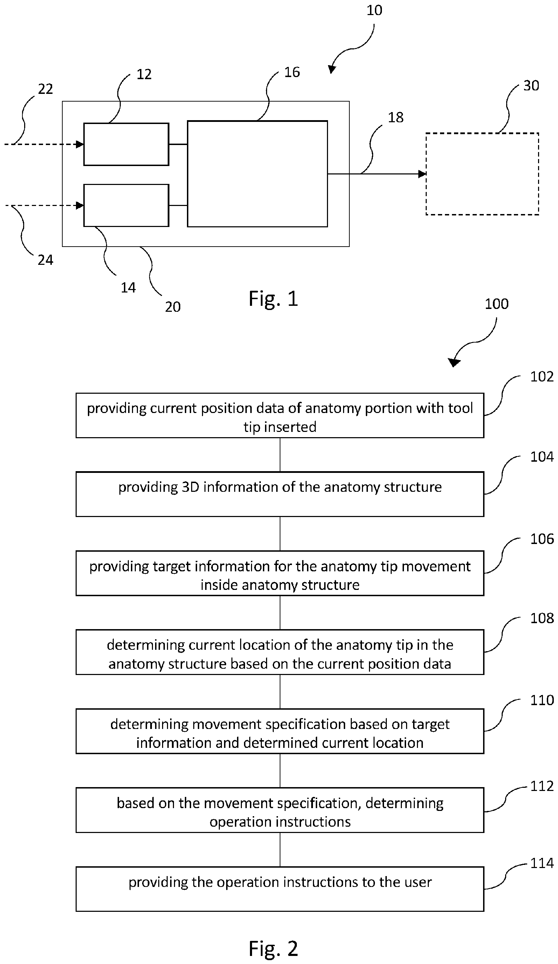

Fig. 1 schematically shows an example of a device 10 for in-body navigation operation instructions. The device comprises a position data supply 12, an anatomical data supply 14, a processor 16 and an output 18.

The position data supply 12 is configured to provide current position data of a tool portion with a tool tip inserted in an anatomy structure in a region of interest of a subject. The anatomical 3D data supply 14 is configured to provide 3D information of the anatomy structure. The anatomical 3D data supply 14 is also configured to provide target information for the tool tip movement inside the anatomy structure. The processor 16 is configured to determine a current location of the tool tip in the anatomy structure based on the current position data. The processor 16 is also configured to determine at least one of the group of movement specification and trajectory information based on the target information and the determined current location in order to align the movement of the tool tip with the target information. The processor 16, based on the at least one of the group of the movement specification and the trajectory information, is further configured to determine operation instructions for the user to achieve a movement of the tool tip according to the at least one of the group of the movement specification and the trajectory information. The output 18 is configured to provide the operation instructions to the user.

In Fig. 1, a frame 20 indicates, as an option, that the position data supply 12, the anatomical data supply 14 and the processor 16 are provided in a common arrangement, for example in a common housing or apparatus. In another option, some or each of the position data supply 12, the anatomical data supply 14 and the processor 16 are provided separately. The output 18 is shown with an arrow to indicate that the determined operation instructions for the user are provided for further purposes like displaying these. In an option, provided in addition or alternatively to visually providing the operation instructions for the user, the operation instructions for the user are output in at least one of the following ways: acoustically and haptically.

In Fig. 1, a first hashed arrow 22 indicates the input of the current position data. A second hashed arrow 24 indicates the input of the 3D information of the anatomy structure. The data input can be provided as live data regarding the input of the current position data. In another example, the input of the current position data is provided from a data storage, for example for test or training purposes when handling a device in a model or simulation. The 3D information of the anatomy structure can be provided by a data storage, such as pre-operational image data. The 3D information of the anatomy structure can also be provided as live data, such as from live imaging.

As a further option, a hashed frame indicates a display 30 that is data-connected to the output 18 (see below). In an example, the display is a monitor. In another example, the display is an image projecting device that projects graphic information on a projection surface, like on a handle of the device. In a further example, the display is provided as an augmented reality display where physical objects are combined, i.e. enhanced, with additional information. For example, the display is provided as a head mounted display for wear by the user. For example, the display is provided as goggles for wear by the user with a display of the additional information, i.e. the operation instructions are provided as projection within the user's view on the reality situation. Thus, a head-up display is provided that comprises the operation instructions to the user.

Fig. 2 shows basic method steps of an example of a method 100 for providing in-body navigation operation instructions. The method 100 comprises the following steps: In a first step 102, also referred to as step a), current position data is provided of an anatomy portion with a tool tip inserted in an anatomy structure in a region of interest of a subject. In a second step 104, also referred to as step b), 3D information of the anatomy structure is provided. In a third step 106, also referred to as step c), target information is provided for the anatomy tip movement inside the anatomy structure. In a fourth step 108, also referred to as step d), a current location of the anatomy tip in the anatomy structure is determined based on the current position data. In a fifth step 110, also referred to as step e), movement specification is determined based on the target information and the determined current location in order to align the movement of the anatomy tip with the target information. In a sixth step 112, also referred to as step f), based on the movement specification, operation instructions for the user to achieve a movement of the anatomy tip are determined according to the movement specification. In a seventh step 114, also referred to as step g), the operation instructions are provided to the user.

The first, second and third steps can be provided simultaneously or in any possible order.

In an example, a method for providing catheterization operation instructions is provided. The method comprises the following steps: providing current position data of a catheter portion with a catheter tip inserted in a vascular structure in a region of interest of a subject; providing 3D information of the vascular structure; providing target information for the catheter tip movement inside the vascular structure; determining a current location of the catheter tip in the vascular structure based on the current position data; determining at least one of the group of movement specification and trajectory information based on the target information and the determined current location in order to align the movement of the catheter tip with the target information; determining, based on the at least one of the group of the movement specification and the trajectory information, operation instructions for the user to achieve a movement of the catheter tip according to at least one of the group of the movement specification and the trajectory information; and providing the operation instructions to the user.

As an example, it is provided the steps of:
- determining movement specification based on the target information and the determined current location in order to align the movement of the anatomy tip with the target information; and
- determining, based on the movement specification, operation instructions for the user to achieve a movement of the anatomy tip according to the movement specification and the trajectory information; and

As another example, it is provided the steps of:
- determining trajectory information based on the target information and the determined current location in order to align the movement of the anatomy tip with the target information;
- determining, based on the trajectory information, operation instructions for the user to achieve a movement of the anatomy tip according to the trajectory information.

An example for in-body navigation is catheterization. Another example for in-body navigation is endoscopy. The term "in-body navigation operation instructions" relates to handling of a steerable tool inserted in a body for which tool navigation is provided.

The tool may be inserted for examining, measuring, imaging or sensing purposes or for performing an intervention.

The term "in-body navigation catheterization operation instructions" relates to handling of a catheter-based intervention, i.e. a procedure, in which a catheter is inserted in a subject. Such procedure is referred to as catheterization.

The term "operation instructions" relates to information and instructions provided to the user e.g. operating the device, like the catheter. Operating can be provided manually and also motorized or at least partly support by a motorized drive. Operating can be provided directly or indirectly. The operation instructions can also be referred to as operation guidance or operational guidance. For example, the operation instructions relate to recommended action(s) to be taken by the user. For example, the operation instructions relate to steering instructions or handling instructions. For example, the operation instructions relate to an operational procedure or manipulation workflow. For example, the operation instructions relate to tool handling, also referred to as steering handling when moving the tool, like moving a catheter inside a vascular structure.

In an example, the tool is a catheter portion with a catheter tip.

In an example, the anatomy structure is a vascular structure, or a cardiac chamber structure or a lumen of another organ. The anatomy structure may also be a tissue region.

The term "position data supply" relates to providing spatial information of a tool., e.g. a catheter tip, i.e. its location in space in order to then bring this into context with the anatomy. The position data supply can also be referred to as position data input or position input.

The term "anatomical data supply" relates to providing spatial information about the anatomy of the region of interest of the subject. The anatomical data supply is also referred to as 3D anatomical data supply or anatomical 3D data supply. The anatomical data refers to three-dimensional information of the vascular structure, i.e. the vessels in the region of interest. The anatomical data supply can also be referred to as anatomical data input or anatomical input.

The term "target information" relates to a target position in the vasculature structure and/or movement information for the tool, e.g. catheter tip, to be followed. The target can thus be a track to follow, such as a particular vessel. The target can thus also be a point or location to which the catheter tip or another type of tool is to be moved. The target information may be manually set or may be determined by further imaging procedures (including AI-driven diagnostic tools), prior or during the intervention.

The target information, for example for a catheter tip movement inside the vascular structure, can also be provided separately, such as by user input.

The anatomical data supply can be provided as a data feed data-connected to a data source. The anatomical data supply can also be provided as data storage data-connected to the processor.

In an option, the position data supply and the anatomical data supply are provided as an integrated supply providing a data input interface. The supply can also be referred to as input or input unit or data input interface.

The term "processor" relates to processing of data. The processor can also be referred to as data processor, data processing arrangement or processor unit. In an example, the processor is data-connected to the position data supply, the anatomical data supply and the user interface.

The "movement specification" relates to the movement that the tool tip, e.g. catheter tip, needs to perform in order to move along according to the target information. The movement specification provides navigation instructions that relate to the path within the vascular structure that is given by the target information.

The "trajectory information" relates to the path, i.e. the planned trajectory that the tool tip, e.g. catheter tip, needs to perform in order to move along that path when approaching the target. The trajectory information provides navigation instructions that relate to the path within the vascular structure as given by the target information.

The term "output" relates to providing the generated operation instructions to the user. The output can also be referred to as output unit. In an example, the output is data-connectable to a user interface like a display arrangement. The user interface can also be referred to as instructor. In an example, the user interface provides the operation instructions in at least one of the following manner: visual way (seeing), tactical way (feeling) and audible way (hearing, listening).

The operation instructions are provided in order to allow a forward motion of the catheter tip, until the target location is reached.

In an example, a device for catheterization operation instructions is provided. The device comprises a position data supply, an anatomical data supply, a processor and an output. The position data supply is configured to provide current position data of a catheter portion with a catheter tip inserted in a vascular structure in a region of interest of a subject. The anatomical 3D data supply is configured to provide 3D information of the vascular structure and to provide target information for the catheter tip movement inside the vascular structure. The processor is configured to determine a current location of the catheter tip in the vascular structure based on the current position data, to determine movement specification based on the target information and the determined current location in order to align the movement of the catheter tip with the target information and, based on the movement specification, to determine operation instructions for the user to achieve a movement of the catheter tip according to the movement specification, The output is configured to provide the operation instructions to the user.

In an example, the anatomical data supply is further configured to provide the data representative of a graphical representation of at least the anatomy structure, e.g. the anatomy structure of the region of interest.

In an example, the data representative of a graphical representation is provided as pre-recorded or pre-determined image data.

In an example, the processor is configured to indicate annotations or tags of the anatomy shown in the image data.

In an example, the data representative of a graphical representation is provided as live images from an image acquisition device.

As an option, the image acquisition device is attached to the tool tip region.

In an example, the image acquisition device is an ultrasound imager, e.g. an IVUS (intravascular ultrasound) sensor.

In an example, the operation instructions also comprise directional information in relation to the catheter tip relating to the vascular structure, like navigation instructions.

In an example, the operation instructions comprise instructions for the user for handling a control handle of a catheter, to which catheter the catheter tip is attached to, which catheter tip is configured to be maneuvered within the vascular structure of the region of interest of a subject. In an example, the operating controls are provided as at least one of the group of: control knobs, adjustment wheels, joystick-like elements, touch controls, buttons on a display and the like.

As an effect, the operating instructions are translated according to the view provided to the user.

Thus, the navigation part is handled by the device itself, and the burden to translate the navigation into actual (manual) action by the user to control the catheter movement is also handled by the device itself. The user can thus simply directly perform the operation instructions.

In an example, in addition or alternatively to visual operation instructions, the use is provided with haptic and tactile feedback while moving the catheter along the vessel structure. For example, an impression is generated on the users side that he/she feels the vessel wall in an improved manner, i.e. in an increased or amplified manner.

The colors, patterns and forms are provided to be distinctable to allow facilitated user information transfer.

In an example, indications like up/down, left/right, front/back, distal/proximal and other anatomy specific indications are not present, neither on the control handle nor in the provided operation instructions

In an example, the output is configured as a user interface that provides the operation instructions to the user and that also enables entry of user commands.

In an example, the control handle interface is configured to receive position data of the handle comprising at least one of the group of orientation in space, location in relation to subject and distance between the control handle and the catheter tip. The processor is further configured to determine the operation instructions based on at least one of the group of orientation in space and location in relation to subject.

The distance between the control handle and the catheter tip influences the amount of elasticity, which is thus taken into account.

The operation instructions are thus provided as context sensitive instructions.

The orientation of the handle is tracked, for example.

The spatial position is provided for augmented reality.

As a further option, the processor is further configured to determine the operation instruction adapted based on a pre-modelled model or measurement-based wire elasticity and stretch. In an example, a measurement-based solution involves continuous analysis of the orientation and angular rotation sensing in both the catheter tip (possibly via imaging) and the control handle, and modelling of the catheter tip rotational response to the control handle's rotational input.

In an example, a turn of the handle of 180° results in only a 90° turn of the tip due to elasticity of the wire shaft. Depending on the length of the part outside the body, this different moving behavior is taken into account for determining the operation instructions.

In an example (not further shown), the operation instructions are provided as augmented reality by overlaying the instructions on an image of the control handle.

In an option, in addition to the operation instructions, navigation information is indicated, wherein the operation instructions are decoupled from the navigation information.

In an example, at least one of the group of operation instructions and navigation information is provided as augmented reality overlaid to a live image of the current situation. As an option, the operation instructions are overlaid onto a physical control handle of the tool.

In an example, the navigation provides indications of at least one of the group of movement paths, movement directions, movement speed, turning points, bifurcations, curves and the like. The navigation information thus relates to the spatial movement along the target path. Contrary, the operation instructions provide instructions for a user how to operate a catheter in order to achieve a movement that is then in accordance with the navigation information.

In an example, the anatomical data supply is further configured to provide data allowing at least two different views on the anatomy structure of the region of interest. The processor is configured to generate image data showing the tool tip in a different view dependent on at least one of the group of the operation instructions, and the complexity of approaching junctions but.

The term "different view" relates to a change of at least one of the group of viewing direction, viewing angle and viewing resolution.

For example, the user, i.e. an operator, can switch from a neutral outside view to a view from the standpoint of the catheter tip. In an example, an automatic change is provided.

In an example, for achieving the target information, a start or entry location for the catheter movement and a target or end location are determined and the processor calculates at least one route for moving the catheter from the entry location to the target location. In an option, different routes are selectable depending on other parameters like geometric and physical properties of the catheter and the catheter tip.

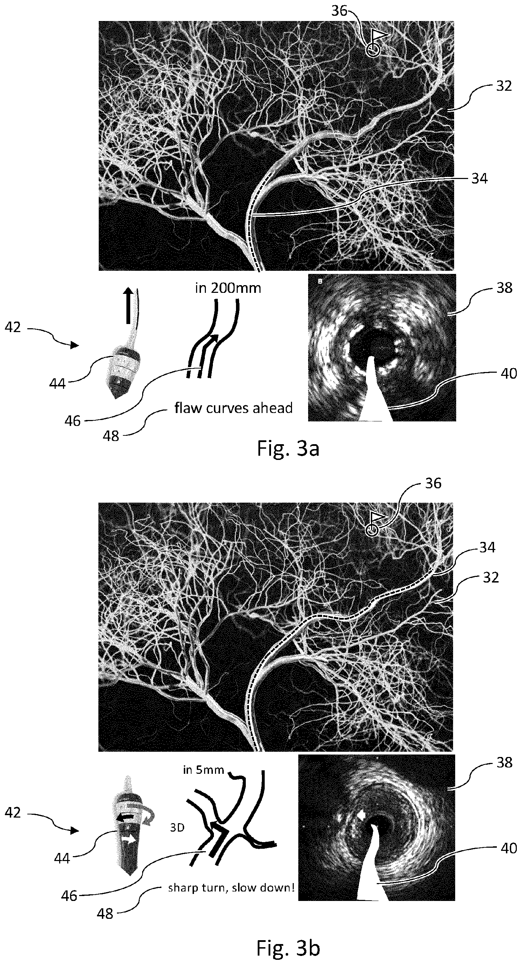

Fig. 3a and Fig. 3b show examples of in-body navigation operation instructions presented to a user. An angiogram 32 or another representation of a vascular structure or another anatomy structure is shown in the upper half of Fig. 3a and Fig. 3b. A partly inserted device 34 is shown and a target point 36 is indicated. An additional image 38 of the inside of the vessel is shown in the lower right parts in Fig. 3a and Fig. 3b. An indication 40 of the target travel path may be provided. The additional image 38 may be an ultrasound image. The lower left parts of Fig. 3a and Fig. 3b shows operating instructions 42, for example in form of a handle 44 with indicated operating directions relating to the handle 44. In addition, also map-like navigation information 46 can be provided, as well as navigation instructions 48 provided as text.

The representation of a vascular structure is provided for an overview. In addition (not shown), a predetermined route, i.e. path within the vascular structure to the target point 36 may be indicated. The representation of the vascular structure thus provides a sort of roadmap.

The additional image 38 provides a personal point of view for the user as if he/she was steering the device within the vessel. The additional image 38 thus provides a cockpit view as seen from the tip of the device. The indication 40 of the of the target travel path provides further understanding of the movement direction. Instead of the indication 40 of the target travel path, the indication may also present the distal end of the device.

The map-like navigation information 46 provides abstract navigation information to the user in relation to the shown parts of the map. The map-like navigation information 46 thus provides a first source information to the user on where the movement has to be steered to. However, this navigation is provided in relation to the map and needs to be transformed by the user into the reference of the steering handle.

The operating instructions 42 provide such information in the context of the handling device, for example the handle 44. The operating instructions 42 thus provide intuitive and direct instructions for a user how to perform the operation of the handle in order to achieve the desired navigation through the vascular structure to arrive at the target point in the desired way along the desired path. The operating instructions 42 are thus provided within the reference of the steering handle and the user can directly follow these.

Fig. 3a shows a situation that is rather easy to steer, as indicated by a straight green arrow (shown in black in Fig. 3a) above the handle 44. The current vessel part is only slightly bended without bifurcation. The provision of the operation instructions allows the user to move forward with the tip of the device.

Fig. 3b shows a situation that requires more steering attention, as indicated by a larger blue turning-to-the-right (in the image) arrow (shown in grey in Fig. 3b) and a smaller red arrow (shown in black in Fig. 3b) for actuating a first control knob to the left and a smaller orange arrow (shown in white in Fig. 3b) for actuating a second control knob to the right. The current vessel part has a complex bifurcation and the target path is following to the left. The provision of the operation instructions allows the user to maneuver though this situation.

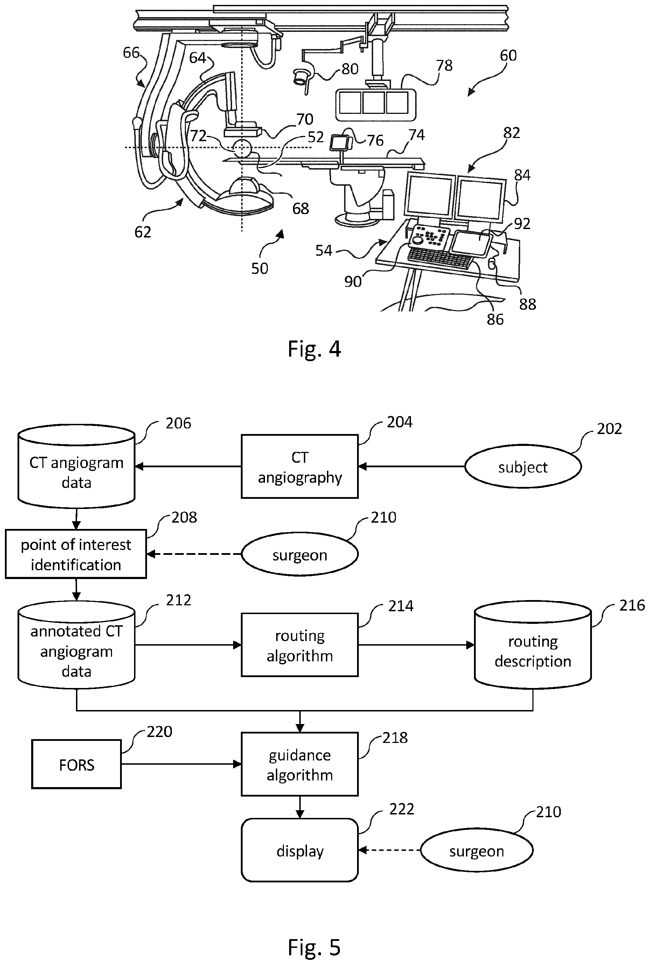

Fig. 4 shows an example of a catheter system 50. The catheter system 50 comprises a catheter 52 having a not further shown control handle, a catheter body and a catheter tip. The catheter system 50 further comprises an example 54 of the device 10 for catheterization operation instructions according to one of the preceding examples. The catheter tip is attached to one end of the catheter body and the catheter control handle is connected to the catheter body towards the other end of the catheter body. The operation instructions comprise instructions for user handling of the control handle in order to maneuver the catheter tip when inserted in a vascular structure of a region of interest of a subject.

In an option, the catheter is equipped with an integrated optical fiber, and a laser is provided to send laser light into the fiber to be reflected back along the fiber for analysis to reconstruct and visualize a full shape of the catheter.

This is based on the concept of measuring strain in optical fibers, using light reflected from density fluctuations in these fibers. This technology, called FORS (Fiber Optic Real Shape) enables real-time 3D visualization of the full shape of catheters inside the body, without the need for continuous imaging, e.g. X-ray imaging. The catheter can be shown in the context of the patient's anatomy through integration with images obtained by pre- or intra- operative techniques (CT, MRI or X-ray fluoroscopy). Clinicians can therefore see with clarity where and how they need to navigate and position their devices within the anatomy.

This supports surgeons who need to see devices in 3D, in real time, in relation to the anatomy, while at the same time reducing exposure to radiation for their patients, their staff and themselves. FORS also enables visualization in multiple, user controlled, unrestricted viewing angles in context of the patient's anatomy (using overlays from preoperative 3D anatomical data or intra-operative X-ray imagery). The FORS system provides 3D information, to de-risk the procedure in terms of potential vessel damage, and to reduce procedure time, since surgeons can navigate the anatomy with more confidence and efficiency. The 3D-shaped catheter can be shown superposed with CT/MRI/X-ray images of the subject. Clinicians are therefore provided with further improved visualization where and how they need to navigate and position their devices within the anatomy.

In an example, a map of the vascular anatomy is created using imaging, e.g. CT or /X-ray angiography.

Fig. 4 shows a cath lab 60 as an example. The cath lab 60 comprises an imaging system, for example an X-ray imaging system 62 with a C-arm 64 movably supported by a ceiling mount 66. An X-ray source 68 and an X-ray detector 70 are provided to image a subject 72 arranged on a subject support 74. A control and display interface 76 are provided next to the subject support 74. Further, a monitor arrangement 78 is indicated. Still further, lighting 80 is also provided.

The example 54 of the device 10 for catheterization operation is provided in the context of a work station or console 82. The console 82 comprises displays 84, a keyboard 86 and a mouse 88. Further, a control console 90 and a graphical tablet 92 are indicated.

In an example, the surgeon points or identifies the starting point, e.g. the groin or the arm of a subject, the destination or point of interest, e.g. towards the heart.

In an example, a vascular navigation system calculates a route from the start point to the point of interest. It is possible to opt for the system to calculate alternative routes to the principal route, e.g. to take into account the size and bending capabilities of a catheter, the size of the implant to be delivered, safety versus efficiency trade-offs, etc. This provides the surgeon with the option of choosing the best route for themselves in that given moment, this way overriding the default criteria used by the system to generate the principal route.

In an example, the surgeon feeds the catheter into the blood vessels and makes the turns based on real-time guidance provided by device: distance to upcoming junctions, next 3D turns to take, recommended speed of advancement, and so on. The device may inform the surgeon about the speed of movement when complex vascular sections are reached, e.g. to slow down in complex vascular sections and to speed up when not in very complex vascular sections.

When in a complicated environment, the device for catheterization can give instructions that are more detailed. In an example, the device for catheterization operation instructions, also referred to as vascular steering support device, provides detailed instructions about which branch at a junction to take.

As a fundamental difference over e.g. a car navigation system, the device for catheterization operation instructions provides instructions to a surgeon about which controls on the catheter control to operate, in what way, and by how much to steer the catheter safely. The device may provide audio, visual and haptic cues for the surgeon on what to do at a certain point on the route.

As indicated above, the device may change the view setting of the imaging device at the tip of the catheter (e.g. ICE, IVUS, OCT) dependent on the vessel shape currently being navigated: At more-or-less straight parts the image settings may have a deeper and more forward looking view setting, while when approaching a junction, the real-time view of the anatomy may switch to an optimal 3D view or 2D plane to visualize the anatomical structure in relation to the device and the branch to navigate to. This offers the possibility to opt for a more suitable, relevant view at the tip of the catheter in order to make the surgeon's movements more intuitive. Some viewing settings offer a better view than other settings depending on the exact location the tip is at that moment. These settings can also help in better estimating device-to-anatomy distance, which makes it more intuitive for the operator (surgeon) to steer the catheter through junctions. The images can be segmented into 3D models to render the vascular feature in more useful way to support navigation.

Fig. 5 shows a further example of a workflow, i.e. procedure, in relation with providing in-body navigation operation instructions.

As preparation, a subject 202 is undergoing an imaging procedure to generate CT angiography 204. This data is further processed to an angiogram, e.g. CT angiogram 206 of the subject and the results are stored in a data storage element. A surgeon 210 annotates this data by identifying 208 the point of entry into the vascular anatomy and the point of interest where diagnostic or interventional procedures have to be performed. The annotated data, also referred to as annotated CT angiogram data 212 is stored in the data storage element. A routing algorithm 214 reads the annotated angiogram data from the data storage element and calculates a route from the point of entry in the vascular anatomy to the point of interest. The routing algorithm can make optimizations based on the individual anatomical variances, the capabilities of the catheter e.g. the bending capability of the catheter tip, and the size of the catheter. The routing algorithm can also take into account the folded implant that the catheter will deliver, e.g. its size. The routing results in routing description 216. The surgeon can start the operation: A guidance algorithm 218 takes inputs from the live catheter FORS data 220, as an example, the annotated angiography data and the description from the storage element. The guidance is displayed in frame 222. The guidance algorithm can further calculate the position of the catheter in the vascular anatomy by overlaying the angiograph data, the routing description data and the FORS data. When a junction in the anatomy is reached, the guidance algorithm can show a 3D representation of the junction, and the position and orientation of the catheter within the anatomy, with an indication of which exit to take. Next, the guidance algorithm calculates cues for the surgeon to maneuver the catheter in the anatomy. When further away from the point of interest and on less complicated sections of the anatomy, the guidance algorithm may guide the surgeon that it is safe to feed the catheter at a higher speed - a safe speed may be calculated from the wideness and curvatures of the vasculature ahead (from angiogram) in relation to catheter size and tip position. Catheter insertion speed can be monitored from the FORS shape in relation to angiogram. This can enable quantitative speed guidance, with warnings if the feeding is going too fast. When closer to the point of interest or more complicated sections of the anatomy, the algorithm gives cues to the surgeon to slow down. When a junction in the vascular anatomy is reached, the algorithm uses the FORS data to calculate the translation and clocking, yaw and pitch maneuvers to point the catheter into the right branch. The angle of bending for the catheter tip is calculated. The surgeon 210 is informed which controls on the catheter handle to actuate and by how much, to safely navigate to the point of interest. This can be achieved by providing light signals on the catheter control handle, augmented reality projections on the control handle (via AR glasses worn by the surgeon), indications on a control handle representation on the navigation guidance display, or in any other way. It is noted that the guidance may take account of discrepancies between the control actuation and catheter tip behavior, due to torsion constraints, feed-length of the catheter and so on. For this it may use known catheter properties, simulations, and/or real-time sensing and mapping of control actuation in relation to catheter movement.

Different view setting of the imaging device at the tip of the catheter (e.g. ICE, IVUS, OCT) can be used to have a better real-time view of the anatomy at the complicated sections of the vascular anatomy. These could be a 3D U/S volume, optimal 3D view or 2D plane to visualize device-to-anatomical structure and distance from the vascular structure. The guidance algorithm, based on the position of the catheter in the vascular system, will change these view settings automatically. The cues to the surgeon can be provided through of audio, haptic, video, augmented reality etc. or a combination.

It is noted that the term "subject" may also be referred to as individual. The "subject" may further also be referred to as patient, although it is noted that this term does not indicate whether any illness or disease is actually present with the subject.

In an example, a computer program enabling a processor to carry out the method of the preceding example is provided.

In an example, a computer program or program element for controlling an apparatus according to one of the examples above is provided, which program or program element, when being executed by a processing unit, is adapted to perform the method steps of one of the method examples above.

In an example, *a* computer readable medium having stored the program element of the preceding example is provided.

It is noted that the present invention can be used in any medical field where catheters are used to navigate through the vascular systems, in the context of treating patients through minimally invasive procedures. It can be used in hybrid cath labs, where such procedures are executed, and where additional image guided therapy equipment is present. In the case of X-ray, the invention may be used in Philips cath labs, equipped with for example Azurion or Allura systems, and possibly ICE, IVUS, OCT, FORS and so on. The proposed guidance may also be used in combination with (autonomous) catheter robots, in which a surgeon can overrule if necessary and use the navigation guidance to continue manually.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for in-body navigation operation instructions, the device comprising:
- a position data supply (12);
- an anatomical data supply (14);
- a processor (16); and
- an output (18);
wherein the position data supply is configured: to provide current position data of a tool portion with a tool tip inserted in an anatomy structure in a region of interest of a subj ect;
wherein the anatomical 3D data supply is configured: to provide 3D information of the anatomy structure; and to provide target information for the tool tip movement inside the anatomy structure;
wherein the processor is configured: to determine a current location of the tool tip in the anatomy structure based on the current position data; to determine at least one of the group of movement specification and trajectory information based on the target information and the determined current location in order to align the movement of the tool tip with the target information; and, based on the at least one of the group of the movement specification and the trajectory information, to determine operation instructions for the user to achieve a movement of the tool tip according to the at least one of the group of the movement specification and the trajectory information; and
wherein the output is configured to provide the operation instructions to the user.

2. Device according to claim 1, wherein the tool is a catheter and the tool tip is a catheter tip; and
wherein the anatomy structure is a vascular structure.

3. Device according to claim 1 or 2, wherein data representative of a graphical representation of at least the anatomy structure of the region of interest is provided and the processor is configured to generate image data showing the tool tip in its current position in context of the anatomy structure; and
wherein the output is a display (30) configured to show the image data comprising the tool tip and the anatomy structure in combination with the operation instructions.

4. Device according to claim 1, 2 or 3, wherein the data representative of a graphical representation is provided as live images from an image acquisition device; and
wherein the image acquisition device is attached to the tool tip region.

5. Device according to one of the preceding claims, wherein the operation instructions comprise instructions for the user for handling a control handle of a tool, to which tool the tool tip is attached to, which tool tip is configured to be maneuvered within the anatomy structure of the region of interest of a subject; and
wherein the operation instructions are provided as handling instructions for the user how to operate a plurality of operating controls of the control handle of the tool, wherein the operating controls relate to movement direction of the tip, bending of the tip portion and movement vector of the tip.

6. Device according to one of the preceding claims, wherein the plurality of operating controls is provided with graphic indicators visible at least for the user of the handle;
wherein the operation instructions relate to the graphic indicators; and
wherein for visual differentiation, the graphic indicators comprise at least one of the group of colors, patterns and forms.

7. Device according to one of the preceding claims, wherein it is further provided a control handle interface, which is configured to receive handle information relating to the operating controls of the control handle and to provide same to the processor; and
wherein the processor is further configured to determine the operation instructions based on the handle information.

8. Device according to claim 7, wherein the control handle interface is configured to receive position data of the handle comprising at least one of the group of orientation in space, location in relation to subject, and distance between control handle and tooltip; and
wherein the processor is further configured to determine the operation instructions based on at least one of the group of orientation in space, location in relation to subject, and distance between control handle and tooltip.

9. Device according to one of the preceding claims, wherein the operation instructions are provided as augmented reality by overlaying the instructions on an image of the control handle; and
wherein, in addition to the operation instructions, navigation information is indicated, wherein the operation instructions are decoupled from the navigation information.

10. Device according to one of the preceding claims, wherein the anatomical data supply is further configured to provide data allowing at least two different views on the anatomy structure of the region of interest; and
wherein the processor is configured to generate image data showing the tool tip in a different view dependent on at least one of the group of the operation instructions, and the complexity of approaching junctions but.

11. A catheter system (50), comprising:
- a catheter (52) comprising a control handle, a catheter body and a catheter tip; and
- a device (54) for catheterization operation instructions according to one of the preceding claims;
wherein the catheter tip is attached to one end of the catheter body and wherein the catheter control handle is connected to the catheter body towards the other end of the catheter body; and
wherein the operation instructions comprise instructions for user handling of the control handle in order to maneuver the catheter tip when inserted in a vascular structure of a region of interest of a subject.

12. System according to claim 11, wherein the catheter is equipped with an integrated optical fiber, and wherein a laser is provided to send laser light into the fiber to be reflected back along the fiber for analysis to reconstruct and visualize a full shape of the catheter.

13. A method (100) for providing in-body navigation operation instructions, comprising the following steps:
- providing (102) current position data of an anatomy portion with a tool tip inserted in an anatomy structure in a region of interest of a subject;
- providing (104) 3D information of the anatomy structure;
- providing (106) target information for the anatomy tip movement inside the anatomy structure;
- determining (108) a current location of the anatomy tip in the anatomy structure based on the current position data;
- determining (110) at least one of the group of movement specification and trajectory information based on the target information and the determined current location in order to align the movement of the anatomy tip with the target information;
- determining (112), based on the at least one of the group of the movement specification and the trajectory information, operation instructions for the user to achieve a movement of the anatomy tip according to the at least one of the group of the movement specification and the trajectory information; and
- providing (114) the operation instructions to the user.

14. A computer program enabling a processor to carry out the method of claim 13.

15. A computer readable medium having stored the program element of claim 14.
